# EUROPEAN PATENT SPECIFICATION

(11) **EP 4 373 605 B1**
(45) Date of publication and mention of the grant of the patent: **03.12.2025**
(21) Application number: 22744230.8
(22) Date of filing: 21.07.2022
(51) Int. Cl.: B01F 25/10, B01F 35/21, B01F 35/45, B01F 101/22

(54) **MIXING DEVICE**
MISCHVORRICHTUNG
DISPOSITIF DE MÉLANGE

(30) Priority: 22.07.2021 EP 21382667
(43) Date of publication of application: 29.05.2024
(73) Proprietor: Innoup Farma, S.L., 31110 Noain-Navarra (ES)
(72) Inventor: GÓMEZ MARTÍNEZ, Sara, 31110 Noáin, Navarra (ES); MARTÍNEZ DE BAROJA CORDOVÍN, Natalia, 31110 Noáin, Navarra (ES); AGÜEROS BAZO, Maite, 31110 Noáin, Navarra (ES)
(74) Representative: ABG Intellectual Property Law, S.L.
(86) International application number: PCT/EP2022/070489
(87) International publication number: WO 2023/001964

(56) References cited:
- CH-A- 495 770
- US-A1- 2009 201 760
- US-A1- 2015 266 206
- US-A1- 2016 346 758
- US-A1- 2017 239 629

## Description

### TECHNICAL FIELD OF THE INVENTION

The present invention belongs to the pharmaceutical field and relates to a mixing device, more in particular a mixing device for the production of nanoparticles.

### BACKGROUND OF THE INVENTION

In the sector of drug production in the chemical and pharmaceutical field, a problem present in the scaling of nanoparticles for industrial manufacturing is the need to maintain the physicochemical properties of the nanoparticles and the reproducibility from batch to batch during manufacturing.

In the specific case of clinical and pre-clinical studies at an early development stage, a small amount of product is sufficient and thus, at such small-scale production, it is possible to achieve great reproducibility with appropriately characterized nanoparticles.

However, when large-scale production is required, batch-to-batch reproducibility and polydispersity of the nanoparticles are difficult to control, difficulties which can result in important variations in the physicochemical properties of the nanoparticles in different batches. This may cause batch rejection and increased manufacturing costs.

The variation in the physicochemical properties of the nanoparticles may also induce undesired effects on the users or patients, thus requiring longer time for testing before large-scale industrial production is achieved.

Generally, there are two approaches for nanoparticles manufacturing: the "top-down" approach and the "bottom-up" approach. The top-down approach creates entities of nanometric size from bigger structures by means of techniques such as milling, whereas the bottom-up approach uses smaller components which assemble to form larger functional structures, such as in monomer polymerization.

In the formulation of nanodrugs a variety of processes may be required, such as high speed homogenization, sonication, milling, emulsification, reticulation, organic solvent evaporation, centrifugation, filtration and/or lyophilisation. Thus, manufacture of nanodrugs products does not simply involve addition and mixing of individual components. Instead, well defined industrial production steps, accurate analysis and strict quality control on the obtained products are required, which results in very high manufacturing costs.

US 2009/201760 A1 discloses a vortex mixer. It also discloses a method of obtaining a supersaturated solution or slurry of a reaction product, wherein at least first and second reactants are introduced into the vortex mixer.

### SUMMARY OF THE INVENTION

The present invention solves the aforementioned problems with a mixing device according to claim 1 and a method for producing nanoparticles according to claim 15. The dependent claims define preferred embodiments of the invention.

In a first inventive aspect, the invention provides a mixing device for the production of nanoparticles by means of the mixing of at least two fluids, wherein the mixing device comprises:
a mixing chamber comprising a base and at least one wall,
two inlet conduits configured to feed a fluid into the mixing chamber, each inlet conduit comprising an inlet portion comprising a plurality of inlet openings, the inlet portions being arranged inside the mixing chamber, spaced from each other and with the inlet openings oriented to supply fluid into the mixing chamber substantially tangentially to the inner surface of the wall, and
an outlet conduit comprising an outlet opening, the outlet conduit being configured to be arranged with the outlet opening located in the mixing chamber at a position higher than the position of the inlet openings.

Each of the inlet conduits of the mixing device of the present invention is configured to be connected to a fluid source to feed fluid into the mixing chamber. The inner surface of the base and the inner surface of the at least one wall of the mixing chamber configure an inner volume for receiving said fluids.

During use, different fluids are supplied into the mixing chamber via the inlet conduits. Insertion of the fluids in the mixing chamber substantially tangentially to the inner surface of the wall, through a plurality of inlet openings, causes a gentle stirring of the fluids inside the mixing chamber which promotes their mixing and is especially suited to prepare nanoparticles by nanoprecipitation, without the need of additional stirring means. This provides the advantage of obtaining a simpler device than those of the state of the art which require the action of additional elements to provide a movement of the fluid.

Substantially tangentially will be understood as the insertion of the fluid into the mixing chamber in a direction fulfilling that the tangential component is greater than the other two perpendicular components. In an embodiment wherein the at least one wall of the mixing chamber is shaped substantially as a surface of revolution, said other two perpendicular components of the direction are the radial direction and the axial direction. Tangentially to the inner surface of the wall will be understood as in a direction tangential at the point of the inner surface of the wall closest to the inlet opening.

When the fluid level inside the mixing chamber, containing the desired product, overflows the outlet opening, the formed nanoparticles enter the outlet conduit and leave the mixing chamber. Nanoparticles can be thus obtained in a continuous process wherein the inlet of the fluids is maintained whilst the outlet provides the formed product, being also able to control the process flow of both raw material entering the mixing chamber and final product when the required reaction is produced.

Discharge by overflowing the outlet opening allows extracting the nanoparticles without applying any force on them. This is advantageous since applying force on the nanoparticles, such as by pumping, may drastically affect their properties, especially in the case of porous nanoparticles. Also, discharge by overflowing the outlet opening allows manufacturing any quantity of nanoparticles, from small to huge volumes, the quantity depending on the time the manufacturing process is maintained in the mixing device of the invention.

The nanoparticle size can be adjusted by controlling the pressure and flow rate of the fluids fed into the mixing chamber. When larger nanoparticle size is required, lower flow rates and pressures are used for the fluids, compared to the case when smaller nanoparticle size is desired. Such adjustment is produced by means of the configuration of the inlet conduits which provide the primary fluids into the mixing chamber.

The mixing device of the present invention allows manufacturing nanoparticles by nanoprecipitation in a continuous process, which is appropriate both for laboratory-scale and industrial-scale production. Said continuous process is achieved by means of the aforementioned configuration, which provides separate inlets for the required fluids, as well as an adequate outlet for the product obtained, such outlet being configured to extract said product from the inner space of the mixing chamber, where the mixing of the raw materials from the inlets occurs, said product being nanoparticles obtained by the mentioned nanoprecipitation procedure.

In an embodiment, to prepare nanoparticles by nanoprecipitation with the mixing device of the present invention, two miscible solvents are used, namely a first solvent which is a good solvent for the material that will form the nanoparticles and a second solvent which is an anti-solvent for said material. The material that will form the matrix of the nanoparticles (e.g. a polymer, protein or any other compound or combinations thereof) is dissolved in the first solvent (e.g. an organic solvent), forming a solvent phase. The first solvent and the second solvent are fed into the mixing chamber, each via one inlet conduit. When the solvent phase is mixed with the anti-solvent (e.g. water), the solvent tends to diffuse into the anti-solvent, causing desolvation of the material and subsequent collapse of the material to form nanoparticles. When the solvent phase includes an active ingredient, said active ingredient is encapsulated in the nanoparticles.

The mixing device of the present invention allows cost-effective and reliable industrial production of nanoparticles by the aforementioned nanoprecipitation, where batches of different nanoparticle sizes can be manufactured, with batch-to-batch reproducibility in a continuous process.

In an embodiment, the inlet conduits are arranged to supply fluid into the mixing chamber in the same direction, i.e. clockwise or anticlockwise. Advantageously, a stirring movement of the fluids is created by means of such inclusion of the fluids inside the mixing chamber which does not induce turbulence in excess in the space available for the formation of the nanoparticles. Thus, the movement produced by the orientation of the inlet conduits, which direct the flow of fluid inside the mixing chamber, is sufficient to promote the formation of nanoparticles, without achieving a fluid movement that deteriorates the obtained product.

In an embodiment, at least a portion of the outlet conduit is arranged along a longitudinal axis of the mixing chamber, i.e. along the direction of the longest dimension of the mixing chamber, which advantageously allows the optimization of the space available inside the chamber for the controlled movement of the fluids introduced therein and the formation of nanoparticles from said movement of the fluids.

In an embodiment the longitudinal axis of the mixing chamber is arranged in the gravity direction.

In an embodiment, the outlet conduit is located at a central position in the mixing chamber. According to this embodiment, the inlet conduits are arranged to supply fluids into the mixing chamber tangentially to the inner surface of the wall, thus surrounding the outlet conduit. This embodiment allows improved mixing of the fluids introduced inside the mixing chamber with the stirring required to induce the formation of nanoparticles and subsequent overflow of the outlet, located in the mixing chamber in an equidistant position from the inner surface of the wall.

In an embodiment, each inlet conduit is arranged with the plurality of inlet openings distributed along a direction substantially parallel to a longitudinal axis of the mixing chamber.

In an embodiment, the inner surface of the at least one wall of the mixing chamber is shaped substantially as a surface of revolution. Preferably, the inner surface of the wall of the mixing chamber is substantially shaped as a cylinder, thus providing a cylindrical chamber together with the base.

In an embodiment, the outlet conduit is arranged along the axis of rotation of the surface of revolution or along a parallel thereof. Preferably, in this embodiment the axis of rotation is the longitudinal axis of the mixing chamber.

In an embodiment wherein the inner surface of the at least one wall of the mixing chamber is shaped substantially as a surface of revolution, each inlet conduit is arranged with the plurality of inlet openings distributed along a direction substantially parallel to the axis of rotation of the surface of revolution. Preferably, in this embodiment the axis of rotation is the longitudinal axis of the mixing chamber.

In an embodiment, the distance from the outlet opening to the base of the mixing chamber is adjustable. The adjustment of the distance from the outlet opening to the base can be achieved in different ways. In an embodiment the outlet conduit is movable relative to the mixing chamber to adjust the position of the outlet opening inside the mixing chamber. Alternatively or additionally, in an embodiment the outlet conduit has an adjustable length, which allows the outlet opening to be located at a desired distance relative to the base of the mixing chamber. Adjusting the distance of the outlet opening from the base of the mixing chamber allows precise positioning of the outlet opening relative to the inlet openings, which has an effect on the movement of fluid inside the mixing chamber, as well as the control of the overflow of the obtained product in order to control the manufacturing process and the batch volume produced. Also, the available volume inside the mixing chamber is dependent on the volume occupied by the outlet conduit and affects the physicochemical properties of the nanoparticles.

In an embodiment, the inlet conduits are arranged spaced from the wall.

In an embodiment, one inlet conduit is arranged at a location of the wake of the other inlet conduit when the mixing device is in operative manner. According to this embodiment, each inlet conduit stands in the way of the flow of fluid introduced in the mixing chamber through the other inlet conduit, improving mixing of both fluids.

In an embodiment, each inlet opening comprises a spraying nozzle having a nozzle opening.

In an embodiment, each spraying nozzle comprises size adjusting means to adjust the size of the nozzle opening. Advantageously, regulation of the size of the nozzle opening allows controlling the stirring effect created inside the mixing chamber by means of the pressure with which the fluid is introduced inside the mixing chamber, as well as adapting the size of the nozzle opening to specific flow rates and/or to the fluids used. Also, this embodiment allows closing one or several of the inlet openings of an inlet conduit to allow the ingress of fluid into the mixing chamber only through selected inlet openings, which provides a better control of the parameters defining the manufacturing process of nanoparticles.

In an embodiment, each inlet conduit comprises a flowmeter.

In an embodiment, the mixing device comprises a cover configured to couple to an upper portion of the mixing chamber to close the inner volume defined by the mixing chamber.

In an embodiment, the mixing device comprises a supporting structure configured to support the mixing chamber. Said supporting structure allows locating the mixing chamber in an adequate location for collecting the produced nanoparticles. In an embodiment the supporting structure comprises a plurality of supporting legs. Preferably, the supporting legs comprise levelling means for improved stabilization of the mixing device.

The invention also provides a mixing system comprising a mixing device according to any of the embodiments according to the first inventive aspect.

In an embodiment the mixing system comprises a collecting chamber in fluid communication with the mixing chamber via the outlet conduit. Preferably, the collecting chamber is arranged below the mixing chamber, to receive the nanoparticles from the mixing chamber produced therein.

In an embodiment the mixing system comprises a bent portion coupled to the outlet conduit and configured to provide fluid communication with the collecting chamber.

In an embodiment the mixing device comprises sealing means configured to seal the coupling between the outlet conduit and the bent portion, such that the flow of the product obtained from the mixing chamber is correctly derived to the collecting chamber.

In an embodiment the mixing system comprises two reservoirs, each connected to an inlet conduit. In the reservoirs the required fluids are stored to be supplied to the mixing chamber by means of the mentioned inlet conduits. Further control of the manufacturing process can be performed by means of the control of reservoir levels and of the provision of the fluids into the mixing chamber.

In an embodiment the mixing system comprises pumping means to supply at least one fluid to the mixing chamber. In a particular embodiment, the pumping means are disposed such that they are able to extract fluid from a reservoir, passing through the inlet conduit with a predetermined pressure and speed, thus controlling the flow rate of such fluid into the mixing chamber.

In a second inventive aspect, the invention defines a method for producing nanoparticles using the mixing device according to any of the embodiments of the invention or the mixing system according to any of the embodiments of the invention, the method comprising the following steps:
(a) providing a first solvent containing the material that will form the nanoparticles and an active ingredient;
(b) providing a second solvent, the second solvent being an anti-solvent for the material that will form the nanoparticles;
(c) supplying the first solvent into the mixing chamber via a first inlet conduit and supplying the second solvent into the mixing chamber via a second inlet conduit;
(d) allowing the fluid level inside the mixing chamber to overflow the outlet opening, such that the formed nanoparticles enter the outlet conduit and leave the mixing chamber.

In an embodiment the first solvent and/or the second solvent are supplied into the mixing chamber using pumping means.

In an embodiment the material that will form the nanoparticles is or comprises a polymer, protein or any other compound of combinations thereof.

In an embodiment the first solvent is an organic solvent.

In an embodiment the second solvent is water.

All the features described in this specification (including the claims, description and drawings) and/or all the steps of the described method can be combined in any combination, with the exception of combinations of such mutually exclusive features and/or steps.

### DESCRIPTION OF THE DRAWINGS

These and other characteristics and advantages of the invention will become clearly understood in view of the detailed description of the invention which becomes apparent from a preferred embodiment of the invention, given just as an example and not being limited thereto, with reference to the drawings.
Figure 1 shows a front view of a mixing device according to an embodiment of the present invention and a detailed view.
Figure 2 shows a lateral view, partially sectioned, of the mixing device of figure 1.
Figure 3 shows a front view of the mixing device of figure 1, with the cover removed.
Figure 4 shows a top section view of the mixing chamber of the mixing device of figure 1.
Figure 5 shows detailed views of an inlet conduit according to an embodiment of the invention.
Figure 6 shows a top view of the mixing device of figure 1.
Figure 7 shows a block diagram of a mixing system according to the present invention.

### DETAILED DESCRIPTION OF THE INVENTION

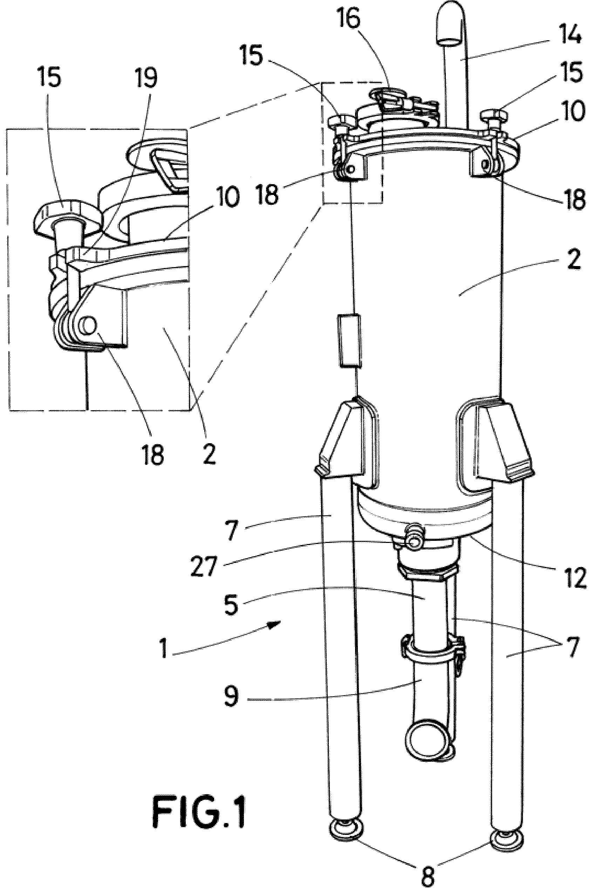

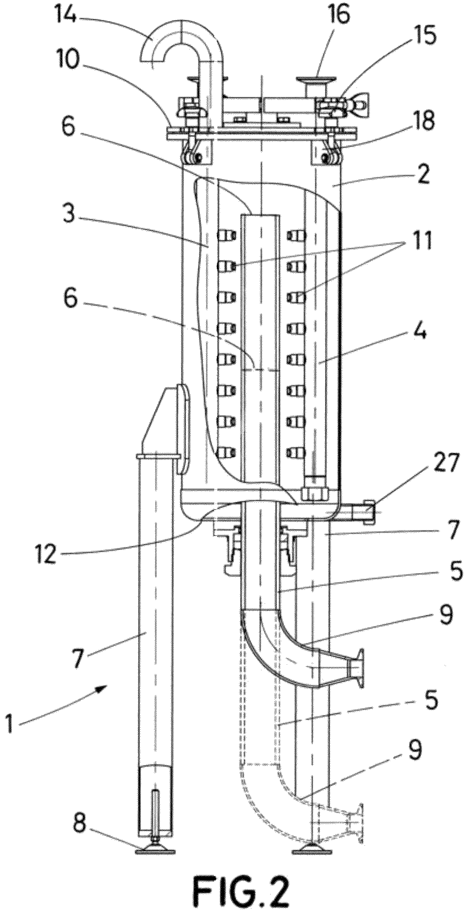

Figures 1 and 2 respectively show a front view and a partially sectioned lateral view of a mixing device according to an embodiment of the present invention. The mixing device (1) comprises a mixing chamber (2), two inlet conduits (3, 4) and an outlet conduit (5).

The mixing chamber (2) comprises a base (12) and at least one wall (13). The inner surface of the base (12) and the inner surface of the at least one wall (13) of the mixing chamber (2) define an inner volume configured for receiving the fluids entering from the two inlet conduits (3, 4). In the embodiment shown, the mixing chamber (2) comprises one single wall (13) with an inner surface substantially shaped as a surface of revolution, namely with a substantially cylindrical shape, thus configuring the mixing chamber (2) as a substantially cylindrical chamber. However, in other embodiments the mixing chamber (2) may have several wall portions and/or may be configured with a different shape. In this embodiment, the mixing chamber (2) is open at its upper end, i.e. the end opposed to the base (12), such that the inner volume of the mixing chamber (2) is accessible.

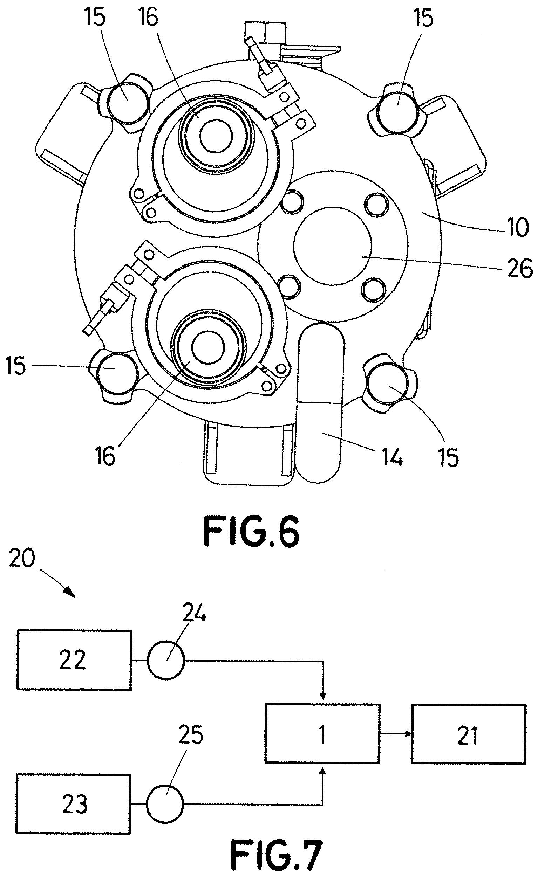

The inlet conduits (3, 4) are configured to be connected to a fluid source, such as a reservoir or deposit (22, 23) schematically depicted in figure 7, and to feed fluid into the mixing chamber (2). Each inlet conduit (3, 4) comprises an inlet portion comprising a plurality of inlet openings (11). As shown in figure 2, in this embodiment the inlet openings (11) are substantially aligned along a portion of the inlet conduits (3, 4). During use of the mixing device (1), the inlet portions of the inlet conduits (3, 4) containing the inlet openings (11) are arranged in the mixing chamber (2) spaced from each other and with the inlet openings (11) oriented to supply fluid into the mixing chamber (2) substantially tangentially to the inner surface of the wall (13). In this embodiment, the inlet conduits (3, 4) are arranged to supply fluid into the mixing chamber (2) in the same direction, namely anticlockwise. Also, in this embodiment, the inlet conduits (3, 4) are arranged with the plurality of inlet openings (11) distributed along a direction substantially parallel to the axis of rotation of the substantially cylindrical mixing chamber, said axis of rotation being also the longitudinal axis of the mixing chamber (2) and being arranged, in use, substantially in the gravity direction. In this embodiment, the inlet conduits (3, 4) are arranged spaced from the wall (13).

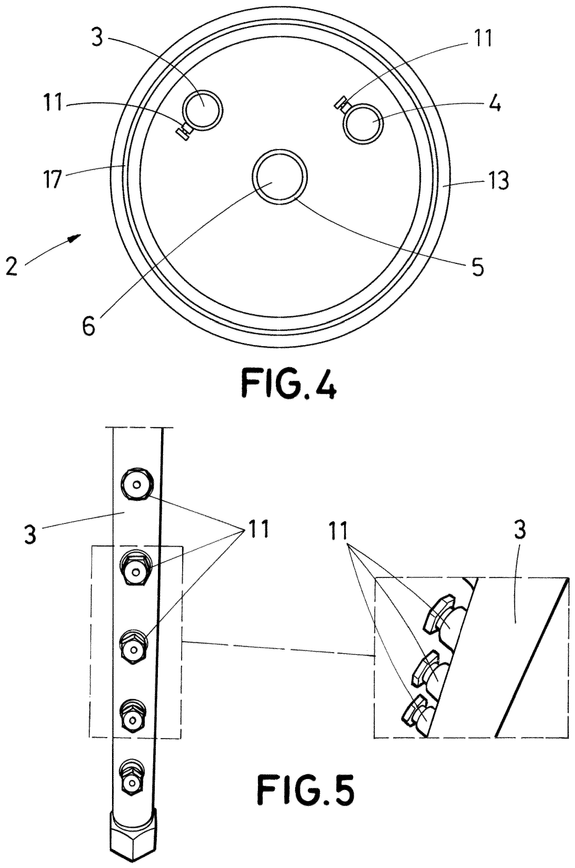

This arrangement of the inlet conduits (3, 4) is schematically shown in figure 4, which shows a top section view of the mixing chamber (2) with the present configuration of the inlet conduits (3, 4).

The outlet conduit (5) comprises an outlet opening (6) and is configured to be arranged with the outlet opening (6) located in the mixing chamber (2) at a position higher than the position of the inlet openings (11), i.e. at a position higher than the position of the highest inlet opening (11). In this embodiment, the outlet conduit (5) is arranged along a central longitudinal axis of the mixing chamber (2) corresponding to the axis of rotation of the substantially cylindrical mixing chamber, and is movable along said longitudinal axis. This is schematically shown in figure 2, where the outlet conduit (5) is depicted in two different positions, namely a first (upper) position where the outlet opening (6) is located in the mixing chamber (2) at a position higher than the position of the inlet openings (11), and a second (lower) position where the outlet opening (6) is located at a lower height compared to the first position. In figure 2 the second position of the outlet conduit (5) is depicted in dashed line to be distinguished from the first position. Different positions other than the ones depicted are also possible for the outlet conduit (5), e.g. intermediate positions of said outlet conduit (5).

In this embodiment, the base (12) of the mixing chamber (2) has an opening to allow insertion of a portion of the outlet conduit (5). By moving the outlet conduit (5) in the longitudinal direction, the length of outlet conduit (5) inserted inside the mixing chamber (2), and thus the position from which the produced nanoparticles are extracted from the mixing chamber (2) once obtained, can be adjusted. Once the desired length of outlet conduit (5) is placed inside the mixing chamber (2), the outlet conduit (5) is releasably secured and remains coupled to the mixing chamber (2) until subsequent release of the outlet conduit (5) is required. Sealing means are preferably provided to assure sealing in the coupling of the outlet conduit (5) and the base (12) of the mixing chamber (2). The end of the outlet conduit (5) opposed to the outlet opening (6) is located outside the mixing chamber (2) and allows discharging manufactured product from the mixing chamber (2). The mixing device (1) may comprise a bent portion (9) coupled to said end of the outlet conduit (5) to improve discharge of the manufactured product, such as by providing fluid communication with a collecting chamber (21). In other embodiments discharge of the manufactured product is performed directly through the end of the outlet conduit (5) or via other means.

Alternatively or additionally to the outlet conduit (5) being movable relative to the mixing chamber (2) to adjust the position of the outlet opening (6) inside the mixing chamber (2), in an embodiment the outlet conduit (5) has a telescopic portion having an adjustable length. Thus, by adjusting the length of the telescopic portion the outlet opening (6) can be located at a desired distance relative to the base of the mixing chamber (2).

In the embodiment shown, each inlet opening (11) of the inlet conduits (3, 4) comprises a spraying nozzle having a nozzle opening. The spraying nozzles comprise size adjusting means to adjust the size of the nozzle opening. Figure 5 shows detailed views of the inlet conduit according to this embodiment of the invention.

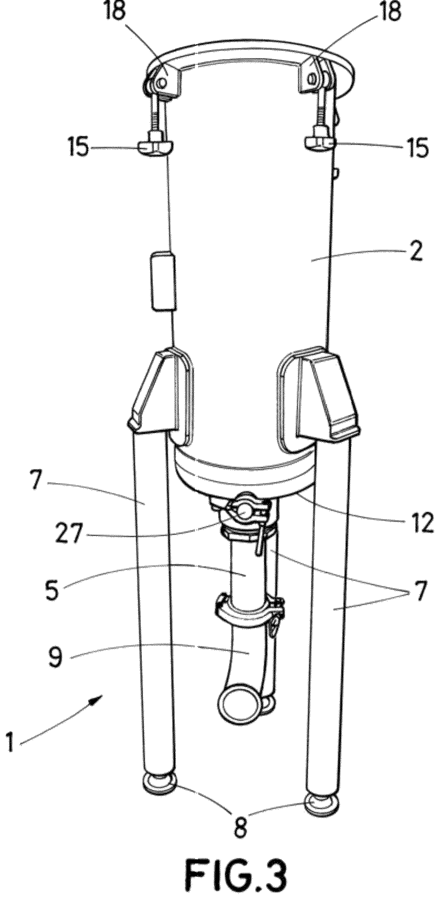

In the embodiment shown, the mixing device (1) comprises a cover (10) configured to couple to an upper portion of the mixing chamber (2) to close the mixing chamber. The cover (10) is visible in figures 1, 2 and 6, whereas figure 3 shows the mixing device with the cover (10) removed. In this embodiment the cover (10) is releasably coupled to the mixing chamber (2) by means of securing bolts (15). The securing bolts (15) are rotatably secured to corresponding lugs (18) arranged at an upper portion of the mixing chamber (2). By rotating the securing bolts (15) from an uncoupled position (shown in figure 3) to a coupled position (shown in figures 1 and 6), the securing bolts (15) are received in slots (19) provided in the cover (10) and the cover (10) is coupled to the mixing chamber (2). The coupled position of the securing bolts (15) is shown in the enlarged detail of figure 1. Additional or alternative securing means between the cover (10) and the upper portion of the mixing chamber (2) are also possible.

Sealing means (not shown) can be provided to seal the coupling between the cover (10) and the mixing chamber (2). In figure 4 a circular groove (17) is visible arranged on a top portion of the mixing chamber (2) for receiving sealing means.

In this embodiment, the cover (10) comprises two openings, through which the inlet conduits (3, 4) are introduced in the mixing chamber (2), such that the inlet portions of the inlet conduits (3, 4) are placed inside the mixing chamber (2). An additional portion of the inlet conduits (3, 4) remains outside the mixing chamber (2) and has a connecting portion (16) for coupling with and receiving fluid from a fluid source. Sealing means can be provided to seal the connection between the inlet conduits (3, 4) and the openings performed in the cover (10).

In this embodiment the cover (10) also includes a window (26) through which the inside of the mixing chamber (2) can be observed even when it is closed by the cover (10).

As shown in figures 1 to 3, in this embodiment the mixing device (1) comprises a supporting structure configured to support the mixing chamber (2). The supporting structure comprises a plurality of supporting legs (7) provided with levelling means (8) for improved stabilization of the mixing device (1) and its placing in suitable locations. The mixing device (1) also includes a bent portion (9) connected to the end of the outlet conduit (5) located outside the mixing chamber, for improved guiding of the obtained manufactured products, i.e. nanoparticles, from the mixing chamber (2) to a collecting chamber (21) where they can be stored or further processed.

In this embodiment the mixing device includes a vent pipe (14) for venting gas from the mixing chamber (2).

In an embodiment the mixing device comprises an outlet port (27) located at a bottom part of the mixing chamber (2). The outlet port (27) comprises closing means for opening/closing the outlet port (27) as required. The outlet port (27) allows easy extraction from the mixing chamber (2) of contents remaining inside said mixing chamber (2) at the end of the process, such as nanoparticles which have not been discharged through the outlet conduit (5) by overflow of the outlet opening (6) or fluids. The outlet port (27) also allows collecting samples during the manufacturing process. Additionally, the outlet port (27) allows the extraction of contents from the mixing chamber (2) in the event of overpressure. This can occur, for example, when the rate of the inlet flow is higher than the rate of the outlet flow through the outlet conduit (5).

Figure 7 shows a block diagram of a mixing system (20) according to the present invention.

Figure 7 schematically shows a mixing system (20) comprising a mixing device (1) according to the invention, a collecting chamber (21), first (22) and second (23) reservoirs and pumping systems (24, 25). Each reservoir (22, 23) is in fluid communication to one inlet conduit (3, 4) of the mixing device (1) and the collecting chamber (21) is in fluid communication with the mixing chamber (2) via the outlet conduit (5). The pumping systems (24, 25) are arranged to supply fluid from the reservoirs (22, 23) to the mixing chamber (2) via the inlet conduits (3, 4).

To prepare nanoparticles with the mixing device of the present invention, two miscible solvents are used, namely a first solvent and a second solvent. The first solvent is a good solvent for the material that will form the nanoparticles and contains the material that will form the nanoparticles and an active ingredient. The second solvent is an anti-solvent for the material that will form the nanoparticles.

The first step is to introduce the first solvent and the second solvent into the first (22) and second (23) reservoir, respectively, which are connected to the mixing chamber by an inlet conduit (3, 4). Then, the solvents contained in the first (22) and second (23) reservoir are pumped into the mixing chamber at a flow rate suitable for the formation of nanoparticles with the desired physico-chemical characteristics. For this purpose, a pumping system (24, 25) is placed between each reservoir (22, 23) and the mixing chamber (2).

The flows of the first solvent and the second solvent are controlled by adjusting the pressure and speed. After adjusting the appropriate flow rates for each solvent, the pumping systems (24, 25) are put into operation and the solvents are introduced into the mixing chamber (2), each solvent via a different inlet conduit (3, 4).

The inlet conduits (3, 4) are arranged with their inlet portions inside the mixing device (1) to supply fluid into the mixing chamber (2) in the same direction (e.g. anticlockwise). A stirring movement of the fluids is created by means of such inclusion of the fluids inside the mixing chamber (2) which does not induce turbulence in excess, sufficient to promote the formation of nanoparticles but without achieving a fluid movement that deteriorates the obtained product.

When the fluid level inside the mixing chamber (2), containing the desired product, overflows the outlet opening (6), the formed nanoparticles enter the outlet conduit (6) and leave the mixing chamber (2), the nanoparticles thus being extracted without applying any force on them.

Finally, the formed nanoparticles are fed to the collecting chamber (21) where they can be stored or processed in a subsequent step.

## Claims

1. A mixing device (1) for the production of nanoparticles from the mixing of at least two fluids, wherein the mixing device (1) comprises:
a mixing chamber (2) comprising a base (12) and at least one wall (13),
two inlet conduits (3, 4) configured to feed a fluid into the mixing chamber (2), each inlet conduit (3, 4) comprising an inlet portion comprising a plurality of inlet openings (11), the inlet portions being arranged inside the mixing chamber (2), spaced from each other and with the inlet openings (11) oriented to supply fluid into the mixing chamber (2) substantially tangentially to the inner surface of the wall (13), and
an outlet conduit (5) comprising an outlet opening (6), the outlet conduit (5) being configured to be arranged with the outlet opening (6) located in the mixing chamber (2) at a position higher than the position of the inlet openings (11).

2. The mixing device (1) according to claim 1, wherein at least a portion of the outlet conduit (5) is arranged along a longitudinal axis of the mixing chamber (2).

3. The mixing device (1) according to any of the previous claims, wherein the distance from the outlet opening (6) of the outlet conduit (5) to the base (12) of the mixing chamber (2) is adjustable.

4. The mixing device (1) according to any of the previous claims, wherein one inlet conduit (3) is arranged at a location of the wake of the other inlet conduit (4) when the mixing device (1) is in operative manner.

5. The mixing device (1) according to any of the previous claims, wherein the inner surface of the wall (13) of the mixing chamber (2) is shaped substantially as a surface of revolution, preferably substantially shaped as a cylinder.

6. The mixing device (1) according to the previous claim, wherein the axis of rotation of the surface of revolution is the longitudinal axis of the mixing chamber.

7. The mixing device (1) according to any of the previous claims, wherein each inlet conduit (3, 4) is arranged with the plurality of inlet openings distributed along a direction substantially parallel to a longitudinal axis of the mixing chamber (2).

8. The mixing device (1) according to any of the previous claims, wherein each inlet opening (11) comprises a spraying nozzle having a nozzle opening, wherein preferably the spraying nozzles comprise size adjusting means to adjust the size of the nozzle opening.

9. The mixing device (1) according to any of the previous claims, further comprising a cover (10) configured to couple to an upper portion of the mixing chamber (2).

10. The mixing device (1) according to any of the previous claims, wherein at least one inlet conduit (3, 4) comprises a flowmeter.

11. A mixing system (20), comprising a mixing device according to any of the previous claims and a collecting chamber (21) in fluid communication with the mixing chamber (2) via the outlet conduit (5).

12. The mixing system (20) according to the previous claim, further comprising a bent portion (9) coupled to the outlet conduit (5) and configured to provide fluid communication with the collecting chamber.

13. The mixing system (20) according to any of claims 11 or 12, further comprising two reservoirs (22, 23), each connected to one inlet conduit (3, 4).

14. The mixing system (20) according to any of claims 11 to 13, further comprising pumping means (24, 25) to supply at least one fluid to the mixing chamber (2).

15. A method for producing nanoparticles using the mixing device (1) according to any of claims 1 to 10 or the mixing system according to any of claims 11 to 14, the method comprising the following steps:
(a) providing a first solvent containing the material that will form the nanoparticles and an active ingredient;
(b) providing a second solvent, the second solvent being an anti-solvent for the material that will form the nanoparticles;
(c) supplying the first solvent into the mixing chamber (2) via a first inlet conduit (3) and the second solvent via a second inlet conduit (4);
(d) allowing the fluid level inside the mixing chamber (2) to overflow the outlet opening (6), such that the formed nanoparticles enter the outlet conduit (6) and leave the mixing chamber (2).

## Patentansprüche

1. Mischvorrichtung (1) für die Herstellung von Nanopartikeln durch das Mischen von mindestens zwei Fluiden, wobei die Mischvorrichtung (1) aufweist:
eine Mischkammer (2) mit einer Basis (12) und mindestens einer Wand (13),
zwei Einlassleitungen (3, 4), die konfiguriert sind, ein Fluid in die Mischkammer (2) einzuspeisen, wobei jede Einlassleitung (3, 4) einen Einlassteil mit mehreren Einlassöffnungen (11) aufweist, die Einlassteile voneinander beabstandet innerhalb der Mischkammer (2) angeordnet sind und die Einlassöffnungen (11) so orientiert sind, um Fluid im Wesentlichen tangential zu der Innenfläche der Wand (13) in die Mischkammer (2) einzuführen, und
eine Auslassleitung (5) mit einer Auslassöffnung (6), wobei die Auslassleitung (5) so konfiguriert ist, dass die Auslassöffnung (6) in der Mischkammer (2) an einer höheren Position angeordnet werden kann, als die Position der Einlassöffnungen (11).

2. Mischvorrichtung (1) nach Anspruch 1, wobei mindestens ein Teil der Auslassöffnung (5) entlang einer Längsachse der Mischkammer (2) angeordnet ist.

3. Mischvorrichtung (1) nach einem der vorstehenden Ansprüche, wobei der Abstand von der Auslassöffnung (6) der Auslassleitung (5) zu der Basis (12) der Mischkammer (2) anpassbar ist.

4. Mischvorrichtung (1) nach einem der vorstehenden Ansprüche, wobei die eine Einlassleitung (3) an einem Ort des Sogs der anderen Einlassleitung (4) angeordnet ist, wenn die Mischvorrichtung (1) im Betriebsmodus ist.

5. Mischvorrichtung (1) nach einem der vorstehenden Ansprüche, wobei die Innenfläche der Wand (13) der Mischkammer (2) im Wesentlichen als eine Drehfläche gestaltet ist, bevorzugt im Wesentlichen als ein Zylinder gestaltet ist.

6. Mischvorrichtung (1) nach dem vorstehenden Anspruch, wobei die Rotationsachse der Drehfläche die Längsachse der Mischkammer ist.

7. Mischvorrichtung (1) nach einem der vorstehenden Ansprüche, wobei jede Einlassleitung (3, 4) so angeordnet ist, dass die mehreren Einlassöffnungen entlang einer Richtung verteilt sind, die im Wesentlichen parallel zu einer Längsachse der Mischkammer (2) ist.

8. Mischvorrichtung (1) nach einem der vorstehenden Ansprüche, wobei jede Einlassöffnung (11) eine Sprühdüse mit einer Düsenöffnung aufweist, wobei die Sprühdüsen vorzugsweise Größenanpassungseinrichtungen aufweisen, um die Größe der Düsenöffnung anzupassen.

9. Mischvorrichtung (1) nach einem der vorstehenden Ansprüche, ferner aufweisend einen Deckel (10), der zum Ankoppeln an einen oberen Teil der Mischkammer (2) konfiguriert ist.

10. Mischvorrichtung (1) nach einem der vorstehenden Ansprüche, wobei mindestens eine Einlassleitung (3, 4) einen Durchflussmesser aufweist.

11. Mischsystem (20) aufweisend eine Mischvorrichtung nach einem der vorstehenden Ansprüche und eine Sammelkammer (21) in Fluidkommunikation mit der Mischkammer (2) via die Auslassleitung (5).

12. Mischsystem (20) nach dem vorstehenden Anspruch, ferner aufweisend einen gebogenen Teil (9), der an die Auslassleitung (5) gekoppelt ist und konfiguriert ist, Fluidkommunikation mit der Sammelkammer bereitzustellen.

13. Mischsystem (20) nach einem der Ansprüche 11 oder 12, ferner aufweisend zwei Vorratsbehälter (22, 23), die jeweils mit einer der Einlassleitungen (3, 4) verbunden sind.

14. Mischsystem (20) nach einem der Ansprüche 11 bis 13, ferner aufweisend Pumpeinrichtungen (24, 25) zum Zuführen mindestens eines Fluids zu der Mischkammer (2).

15. Verfahren zur Herstellung von Nanopartikeln unter Verwendung der Mischvorrichtung (1) nach einem der Ansprüche 1 bis 10 oder des Mischsystems nach einem der Ansprüche 11 bis 14, wobei das Verfahren die folgenden Schritte aufweist:
(a) Bereitstellen eines ersten Lösungsmittels, das das Material, das die Nanopartikel bilden wird, und einen Wirkstoff enthält;
(b) Bereitstellen eines zweiten Lösungsmittels, wobei das zweite Lösungsmittel ein Gegen-Lösungsmittel für das Material ist, das die Nanopartikel bilden wird;
(c) Einführen des ersten Lösungsmittels in die Mischkammer (2) via eine erste Einlassleitung (3) und des zweiten Lösungsmittels via eine zweite Einlassleitung (4);
(d) Erlauben, dass der Fluidpegel in der Mischkammer (2) die Auslassöffnung (6) überströmt, so dass die gebildeten Nanopartikel in die Auslassleitung (6) eintreten und die Mischkammer (2) verlassen.

## Revendications

1. Dispositif de mélange (1) pour la production de nanoparticules à partir du mélange d'au moins deux fluides, dans lequel le dispositif de mélange (1) comprend :
une chambre de mélange (2) comprenant une base (12) et au moins une paroi (13),
deux conduits d'entrée (3, 4) configurés pour introduire un fluide dans la chambre de mélange (2), chaque conduit d'entrée (3, 4) comprenant une partie d'entrée comprenant une pluralité d'ouvertures d'entrée (11), les parties d'entrée étant disposées à l'intérieur de la chambre de mélange (2), espacées l'une de l'autre et avec les ouvertures d'entrée (11) orientées de manière à introduire le fluide dans la chambre de mélange (2) sensiblement tangentiellement à la surface intérieure de la paroi (13), et
un conduit de sortie (5) comprenant une ouverture de sortie (6), le conduit de sortie (5) étant configuré pour être disposé avec l'ouverture de sortie (6) située dans la chambre de mélange (2) à une position plus élevée que la position des ouvertures d'entrée (11).

2. Dispositif de mélange (1) selon la revendication 1, dans lequel au moins une partie du conduit de sortie (5) est disposée le long d'un axe longitudinal de la chambre de mélange (2).

3. Dispositif de mélange (1) selon l'une quelconque des revendications précédentes, dans lequel la distance entre l'ouverture de sortie (6) du conduit de sortie (5) et la base (12) de la chambre de mélange (2) est réglable.

4. Dispositif de mélange (1) selon l'une quelconque des revendications précédentes, dans lequel un conduit d'entrée (3) est disposé à l'emplacement du sillage de l'autre conduit d'entrée (4) lorsque le dispositif de mélange (1) est en fonctionnement.

5. Dispositif de mélange (1) selon l'une quelconque des revendications précédentes, dans lequel la surface intérieure de la paroi (13) de la chambre de mélange (2) est formée sensiblement comme une surface de révolution, de préférence sensiblement en forme de cylindre.

6. Dispositif de mélange (1) selon la revendication précédente, dans lequel l'axe de rotation de la surface de révolution est l'axe longitudinal de la chambre de mélange.

7. Dispositif de mélange (1) selon l'une quelconque des revendications précédentes, dans lequel chaque conduit d'entrée (3, 4) est disposé avec la pluralité d'ouvertures d'entrée réparties le long d'une direction sensiblement parallèle à l'axe longitudinal de la chambre de mélange (2).

8. Dispositif de mélange (1) selon l'une quelconque des revendications précédentes, dans lequel chaque ouverture d'entrée (11) comprend une buse de pulvérisation ayant une ouverture de buse, dans laquelle les buses de pulvérisation comprennent de préférence des moyens de réglage de la taille pour ajuster la taille de l'ouverture de buse.

9. Dispositif de mélange (1) selon l'une quelconque des revendications précédentes, comprenant en outre un couvercle (10) configuré pour s'accoupler à une partie supérieure de la chambre de mélange (2).

10. Dispositif de mélange (1) selon l'une quelconque des revendications précédentes, dans lequel au moins un conduit d'entrée (3, 4) comprend un débitmètre.

11. Système de mélange (20), comprenant un dispositif de mélange selon l'une quelconque des revendications précédentes et une chambre de collecte (21) en communication fluidique avec la chambre de mélange (2) via le conduit de sortie (5).

12. Système de mélange (20) selon la revendication précédente, comprenant en outre une partie coudée (9) couplée au conduit de sortie (5) et configurée pour fournir une communication fluidique avec la chambre de collecte.

13. Système de mélange (20) selon l'une quelconque des revendications 11 ou 12, comprenant en outre deux réservoirs (22, 23), chacun relié à un conduit d'entrée (3, 4).

14. Système de mélange (20) selon l'une quelconque des revendications 11 à 13, comprenant en outre des moyens de pompage (24, 25) pour fournir au moins un fluide à la chambre de mélange (2).

15. Méthode de production de nanoparticules à l'aide du dispositif de mélange (1) selon l'une quelconque des revendications 1 à 10 ou du système de mélange selon l'une quelconque des revendications 11 à 14, la méthode comprenant les étapes suivantes :
(a) fournir un premier solvant contenant le matériau qui formera les nanoparticules et un ingrédient actif ;
(b) fournir un second solvant, le second solvant étant un anti-solvant pour le matériau qui formera les nanoparticules ;
(c) introduire le premier solvant dans la chambre de mélange (2) par un premier conduit d'entrée (3) et le second solvant via un second conduit d'entrée (4) ;
(d) permettre au niveau de fluide à l'intérieur de la chambre de mélange (2) de déborder par l'ouverture de sortie (6), de sorte que les nanoparticules formées pénètrent dans le conduit de sortie (6) et quittent la chambre de mélange (2).
